# FASCICULE DE BREVET EUROPEEN

(11) **EP 2 640 363 B1**
(45) Date de publication et mention de la délivrance du brevet: **26.08.2015**
(21) Numéro de dépôt: 11794834.9
(22) Date de dépôt: 18.11.2011
(51) Int. Cl.: A61K 9/20, A61K 9/28, A61K 9/50, A61K 31/215, A61K 31/717, A61K 31/745, A61K 33/06, A61K 33/10

(54) **COMPOSITION PHARMACEUTIQUE COMPORTANT DES SELS DE CITRATE ET DE BICARBONATE, ET SON UTILISATION POUR LE TRAITEMENT DE LA CYSTINURIE**
PHARMAZEUTISCHE ZUSAMMENSETZUNG MIT CITRAT UND BICARBONATSALZEN UND IHRE VERWENDUNG ZUR BEHANDLUNG VON CYSTINURIE
PHARMACEUTICAL COMPOSITION COMPRISING CITRATE AND BICARBONATE SALTS, AND USE THEREOF FOR TREATING CYSTINURIA

(30) Priorité: 18.11.2010 FR 1059474
(43) Date de publication de la demande: 25.09.2013
(73) Titulaire: Advicenne, 30000 Nîmes (FR)
(72) Inventeur: GUITTET, Catherine, 13200 Arles (FR); GRANIER, Luc-André, 30490 Montfrin (FR); ROUSSEL-MAUPETIT, Caroline, 38330 Saint-Ismier (FR)
(74) Mandataire: Santarelli
(86) Numéro de dépôt international: PCT/FR2011/052697
(87) Numéro de publication internationale: WO 2012/066257

(56) Documents cités:
- EP-A1- 1 970 066
- US-A- 3 903 255
- David S Goldfarb: "Urinary Alkalization", Cystinuria Support Network internet article, 27 septembre 2008 (2008-09-27), pages 1-3, XP002667840, Extrait de l'Internet: URL:http://www.cystinuria.com/articles/uri nary-alkalization/ [extrait le 2012-01-20] & David S Goldfarb: "Urinary Alkalization", http://www.cystinuria.com internet article, 27 septembre 2008 (2008-09-27), Extrait de l'Internet: URL:http://web.archive.org/web/20080927075 845/http://www.cystinuria.com/articles/uri nary-alkalization/ [extrait le 2012-01-20]
- BIYANI C S ET AL: "Cystinuria-Diagnosis and Management", EAU - EBU UPDATE SERIES, ELSEVIER, AMSTERDAM, NL, vol. 4, no. 5, 1 octobre 2006 (2006-10-01) , pages 175-183, XP024992348, ISSN: 1871-2592, DOI: 10.1016/J.EEUS.2006.06.001
- Anonymous: "patient handout potassium bicarbonate and potassium citrate K-lite", Mescape internet article, 1 mai 2010 (2010-05-01), XP002648918, Extrait de l'Internet: URL:http://reference.medscape.com/drug/klo r-con-ef-k-lyte-potassium-bicarbonate-pota ssium-citrate-999539#91 [extrait le 2011-07-08]

## Description

La présente demande concerne une composition pharmaceutique comprenant du sel de citrate et du sel de bicarbonate, utilisable comme médicament en particulier pour le traitement de la cystinurie.

La cystinurie, qui affecte dans le monde une personne sur 7000, est un désordre héréditaire du transport des acides aminés dibasiques que sont les cystine, ornithine, lysine et arginine. Ce désordre du transport se traduit généralement par une élimination urinaire excessive et un trouble de l'absorption intestinale de cystine.

La lithiase cystinique est la seule manifestation clinique de la cystinurie. Elle représente de 1 à 3% des lithiases chez l'adulte et de 6 à 8% des lithiases chez l'enfant.

La gravité de la maladie tient au fait que la cystine est très peu soluble dans les urines, car le défaut d'absorption digestive de ces acides aminés n'a aucune conséquence clinique. La sursaturation des urines en cystine induit la formation de cristaux et des calculs de cystine sont créés. Il n'existe pas d'inhibiteur connu de la cristallisation de cystine.

La solubilité de la cystine dépend du pH des urines. Plus le pH est élevé et alcalin, plus la cystine est sous forme soluble.

L'objectif du traitement médical de la cystinurie est de maintenir les urines sous-saturées en cystine. Cela est effectué généralement par au moins l'une des actions suivantes :
- abaisser la concentration urinaire de cystine par un régime pauvre en méthionine et hyposodé, et par la dilution des urines : un volume d'urine d'au moins 3 litres par jour est nécessaire pour dissoudre la totalité de la cystine. Ce traitement nécessite une répartition régulière des boissons au cours du nycthémère et en particulier la nuit.
- augmenter la solubilité de la cystine par alcalinisation des urines, de façon à maintenir constamment le pH urinaire à des valeurs alcalines (supérieure ou égale à 7.0).

Aucune des différentes actions ne fournit à elle seule une efficacité complète. En cas d'échec, ces actions sont complétées par la prescription d'un composé sulfhydrylé, de préférence la tiopronine.

Différentes compositions à base de citrate ou à base de bicarbonate sont utilisées pour alcaliniser les urines de patients présentant une cystinurie :
- l'eau de Vichy comportant du bicarbonate de sodium mais qui peut exposer à la fluorose ;
- le citrate de potassium assurant la même alcalinisation que le bicarbonate sans augmenter la natriurèse. Il doit être prescrit à la posologie de 6 à 8 g/jour dilué dans 1,5 à 2 litres d'eau. La tolérance gastrique du citrate de potassium est médiocre et la palatabilité des formes pharmaceutiques mauvaise.

L'alcalinisation telle qu'elle est pratiquée actuellement consiste en l'absorption de doses de citrate ou de doses de bicarbonate, de multiples fois par jour et par nuit, du fait de leur courte efficacité.

Ainsi l'alcalinisation par le bicarbonate de sodium à la posologie de 8 à 16 grammes par jour (g/j) chez l'adulte, bien répartis sur le nycthémère dans 2 à 3 litres d'eau, reflète l'une de ses pratiques. De plus fortes doses (30 à 40 g/j) de bicarbonate de sodium permettent théoriquement de maintenir constamment le pH urinaire supérieur à 7,0 voire dans certains cas à atteindre le pH de 7,5 mais leur tolérance gastrique est médiocre.

Les formes pharmaceutiques actuelles sont loin de permettre une alcalinisation optimale, même en cas de bonne observation du traitement. Il est à l'heure actuelle impossible dé maintenir le pH urinaire supérieur à 7 en continu. En effet, les formes pharmaceutiques commerciales supposent une prise toutes les deux heures, y compris la nuit. Cela représente une contrainte très importante et difficilement acceptable sur le moyen ou long terme pour les patients.

En outre, chacune de ces compositions de sels a une faible tolérance gastro-intestinale, ce qui est un autre inconvénient de leur utilisation et limite la dose par prise.

A l'heure actuelle, le seul exemple d'association de ces deux sels dans une formulation unique est le médicament commercialisé sous forme de comprimés effervescents sous le nom de Kalium Hausmann Effervettes® chaque comprimé comprenant 1700mg de citrate de potassium et 1440mg de bicarbonate de potassium, Cette formulation ne résout cependant pas les problèmes exposés ci-dessus puisque les deux principes actifs se libèrent en même temps et de manière immédiate. En particulier la tolérance gastrique de ces comprimés est très mauvaise. En outre, Kalium Hausmann Effervettes® n'est pas indiqué dans la cystinurie mais en cas de déficit potassique.

Le document EP 1970066 A1 décrit l'utilisation d'une composition pharmaceutique comprenant du bicarbonate de métaux alcalins ou alcalino-terreux, pour le traitement de l'acidose métabolique ou de l'acidose conséquence d'une insuffisance rénale chronique. La composition décrite dans ce document comporte un revêtement sous forme de film gastro-résistant (ou entérique), ainsi qu'une matrice pâteuse de matériaux lipophiles comportant le principe actif. Le fait que le revêtement est gastro-résistant signifie qu'à des pH très acides comme ceux de l'estomac, il n'y a pas de libération du principe actif de la composition. Par conséquent, le revêtement de cette composition est pH-dépendant.

Ce document est à rapprocher d'une publication JPP 2007, 59 : 59 - 65, Breitkreutz et al. "Enteric coated solid Dosage forms containing sodium bicarbonate as a drug substance: an exception from the rule?*"*, dont l'un des auteurs M. Peter Kalesh est inventeur de EP 1971666. Cette publication montre que les compositions de type gélules Nephrotrans^{®} ou bicaNorm, ainsi que des compositions de micro-comprimés, présentent une cinétique de dissolution (et donc de libération du principe actif) dépendant du pH. En outre, la figure 4 page 65 de ce document montre qu'à pH de 6,8, qui est le pH de l'intestin grêle, le principe actif est libéré à 100% au bout de 3 heures.

Les formulations ainsi décrites dans ces deux documents répondent au problème spécifique des acidoses, qui sont traitées de façon à apporter une dose massive de bicarbonate dans le sang.

L'article de David S. Godfarb "Urine alkalinization", publié sur le site www.cystinuria.com le 27 septembre 2008, fait une revue des traitements disponibles pour l'alcalinisation des urines d'un patient atteint de cystinurie. En particulier, cet article recommande la prise de citrate ou de bicarbonate. Toutes les formulations citées sont à libération immédiate. En outre, elles posent des problèmes de goût, de mauvaise tolérance gastrique et d'observance. L'ingestion de citrate de potassium est cependant recommandée.

Le document US 3,903,255 décrit un comprimé effervescent à base de chlorure de potassium et de bicarbonate de potassium permettant de fournir du potassium au patient qui en a besoin.

La publication *"*Cystinuria - Diagnosis and management", Biyanic. S. et al. EAU-EBU update séries, Vol. 4, No. 5, October 1, 2006, pp. 175-183 décrit la possibilité de traitement de cystinurie (paragraphe 6.3 pages 178-179). Il décrit l'utilisation du bicarbonate de sodium ou de citrate de potassium.

Le but de la présente invention est donc de proposer une composition qui résout les nombreux problèmes des compositions de l'art antérieur, et qui en particulier permet, par un nombre de prises journalières modéré, par exemple de deux, de maintenir le pH urinaire du patient à une valeur alcaline, supérieure ou égale à 7.

Ainsi la Demanderesse a mis au point: Composition pharmaceutique solide à usage oral sous forme de comprimés comprenant :- une première formulation pharmaceutique solide à usage oral sous forme d'au moins un micro-comprimé, le micro-comprimé ayant une taille comprise dans une fourchette de 2 à 4 mm, et étant constitué d'un coeur comprenant au moins un sel précurseur du cycle de Krebs en tant que principe actif, et d'un enrobage comprenant au moins un agent d'enrobage, le sel précurseur du cycle de Krebs étant choisi parmi les fumarates, les malates, les citrates, l'alpha-cétoglutarate, le succinyl-Coenzyme A, les succinates et l'oxaloacétate, et - une seconde formulation pharmaceutique à usage oral sous forme d'au moins un mini-comprimé, le mini-comprimé ayant une taille comprise dans une fourchette de 2 à 25 mm, et le mini-comprimé étant constitué d'un coeur comprenant au moins un sel de bicarbonate en tant que principe actif et au moins une matrice hydrophile à libération prolongée, et d'un enrobage comportant au moins un agent d'enrobage, la dissolution in vitro de la seconde formulation dans un milieu de dissolution tamponné à pH donné dans une plage entre 1,3 et 7, avec un appareil de dissolution de type 2, conforme à la Pharmacopée Européenne (Ph. Eur.) 2.9.3 « essai de dissolution des formes solides », se produisant selon une cinétique indépendante du pH.

De préférence le micro-comprimé de la première formulation pharmaceutique consiste en un coeur et un enrobage.

De préférence, le mini-comprimé de la seconde formulation pharmaceutique consiste en un coeur et un enrobage.

Les deux formulations pharmaceutiques sont différentes l'une de l'autre. La composition selon l'invention est une composition pharmaceutique alcalinisante, administrable oralement, comportant du sel précurseur du cycle de Krebs, de préférence du sel de citrate, et du sel de bicarbonate, et qui présente des améliorations notables par rapport aux compositions connues de l'art antérieur. Les première et seconde formulations sont administrées simultanément au patient, mais leurs actions pharmacocinétiques et pharmacologiques sont complémentaires et différées, indépendamment, dans le temps.

Un des avantages de la composition de l'invention est sa grande durée d'action. En effet, on a constaté chez trois sujets un maintien du pH urinaire à des valeurs préconisées (pH compris entre 7,0 et 8,0) sur plusieurs heures, et idéalement sur une durée de 8 heures afin de couvrir toute une nuit. Cela évite avantageusement aux patients la contrainte de se réveiller la nuit et d'ingérer une solution alcalinisante afin d'alcaliniser en continu leurs urines.

Un autre avantage de la composition selon l'invention est une meilleure tolérance gastrique, et une adaptation plus facile des doses.

En outre la composition selon l'invention est facile à avaler et d'un goût acceptable.

Enfin, la composition selon l'invention permet d'éviter un matraquage de l'organisme par une surcharge alcaline proximale brutale des principes actifs, en répartissant leur charge progressivement tout au long de l'intestin. Par conséquent l'efficacité de la composition selon l'invention tout au long du nycthémère est meilleure que celle des compositions selon l'art antérieur.

Dans un mode de réalisation préféré, la composition consiste en une première formulation et en une seconde formulation, i.e. qu'elle ne comporte aucun autre composant que ces deux formulations.

De préférence, la composition comprend de 30 à 70% de première formulation et de 70 à 30% de seconde formulation, en poids par rapport au poids total de la composition. A titre d'exemple, la composition comprend 33% de première formulation et 67% de seconde formulation, en poids par rapport au poids total de composition.

Le mélange de micro-comprimés de première formulation et de mini-comprimés de seconde formulation se fait généralement de façon à assurer une répartition homogène de ces deux formulations dans l'ensemble de la composition. Ainsi la composition comprend de préférence une répartition homogène des deux formulations en son sein, c'est-à-dire que, par exemple pour une composition à 50% de première formulation et 50% de seconde formulation, un comprimé choisi au hasard a autant de probabilité d'être un comprimé de première formulation que d'être un comprimé de seconde formulation.

Chacun des micro- et mini-comprimés selon l'invention est enrobé. Selon la définition de la Pharmacopée Européenne (Ph. Eur.), un comprimé enrobé est un comprimé recouvert d'une ou plusieurs couches de mélange de substances diverses telles que résine naturelles ou synthétiques, gommes, gélatine, charges insolubles inactives, sucres, substances plastifiantes, polyols, cires, colorants autorisés par l'Autorité compétente et, parfois, aromatisants et substances actives. Toutefois, selon l'invention, il est exclu que l'enrobage comprenne un principe actif, que ce soit un sel précurseur du cycle de Krebs ou un sel de bicarbonate.

Quand l'enrobage est constitué d'un film polymère très mince, le comprimé est dit pelliculé (cf. Ph. Eur.).

Avantageusement, l'enrobage permet à la fois de masquer le goût et de gérer la cinétique de libération du principe actif contenu dans le comprimé enrobé.

Le « coeur de comprimé » est, selon l'invention, toute la partie du comprimé qui n'est pas l'enrobage.

Par « composition pharmaceutique », on entend selon l'invention une composition dont les composants sont acceptables d'un point de vue pharmaceutique. En particulier, la composition est constituée de composants appropriés et acceptables pour une administration pharmaceutique orale. Par conséquent, chacune de deux formulations pharmaceutiques est elle aussi constituée de composants appropriés et acceptables pour une administration pharmaceutique orale.

Par « composant choisi parmi les éléments », on entend que le composant est un des éléments ou bien un mélange de ces éléments.

La libération contrôlée observée *in vitro* (séparément) à la fois pour la première formulation et pour la seconde formulation, reflète la libération contrôlée dans l'organisme de ces deux formulations, et donc de la composition. Une telle libération est qualifiée de « prolongée » car elle atteint ou dépasse une durée d'une heure.

Cette libération contrôlée observée *in vitro* reflète une libération contrôlée dans l'organisme, qui peut être vérifiée par mesure du pH urinaire des sujets traités par cette composition, usuellement à intervalles réguliers, par exemple toutes les deux heures.

### Première formulation pharmaceutique

Par « sel précurseur du cycle de Krebs », on entend selon l'invention au moins un sel choisi parmi les fumarates, les malates, les citrates, l'alpha-cétoglutarate, le succinyl CoA (ou succinyl-Coenzyme A), les succinates et l'oxaloacétate. Ces sels jouent tous un rôle dans le cycle de Krebs.

Le sel précurseur du cycle de Krebs est de façon particulièrement préférée un sel de citrate.

Le sel de citrate est de préférence choisi parmi le citrate de potassium, le citrate de sodium et le citrate de magnésium, et de façon encore plus préférée le sel de citrate est le citrate de potassium.

La première formulation pharmaceutique selon l'invention permet très avantageusement une libération continue *in vivo* du sel précurseur du cycle de Krebs, après une prise unique, sur une durée généralement de quatre heures au maximum, de façon contrôlée. Par libération continue, on entend selon l'invention une libération qui s'effectue constamment *in vivo*, depuis la prise unique de la composition jusqu'à une durée d'environ quatre heures au maximum.

De préférence, la première formulation pharmaceutique selon l'invention est telle qu'elle libère *in vivo* pratiquement tout le sel précurseur du cycle de Krebs (c'est-à-dire au moins 95% dudit sel) sur une durée maximale d'environ quatre heures après une prise unique de la composition.

Selon une variante préférée, la première formulation selon l'invention est apte à libérer (ou dissoudre) le sel précurseur du cycle de Krebs *in vitro* dans un milieu de dissolution d'eau purifiée à pH 7 réalisé avec un appareil dé dissolution de type 2, conforme à la Pharmacopée Européenne (Ph. Eur.) 2.9.3 « *Essai de dissolution des formes solides* », à un taux de 2 à 15% en 15 minutes, de 15 à 25% en 30 minutes, et de 30 à 50% en une heure.

Ce pH de 7 est une mesure aisée à pratiquer en laboratoire, car c'est le pH de l'eau purifiée. La mesure se fait donc simplement par dissolution dans de l'eau purifiée.

Selon une variante préférée, la première formulation selon l'invention est apte à libérer (ou dissoudre) le sel précurseur du cycle de Krebs *in vitro* dans un milieu de dissolution de solution tamponnée à pH 1,3 réalisé avec un appareil de dissolution de type 2, conforme à la Pharmacopée Européenne (Ph. Eur.) 2.9.3 « *Essai de dissolution des formes solides* », à un taux de 2 à 15% en 15 minutes, de 15 à 25% en 30 minutes, et de 30 à 50% en une heure.

Ce pH de 1,3 est représentatif du milieu acide de l'estomac.

Pour ces mesures, un gramme de première formulation pharmaceutique, ce qui correspondant à une unité de dosage, est placée dans un banc de dissolution de type Pharmatest modèle PTW S3C, dans lequel les conditions de température sont 37°C ± 0,5°C, et la vitesse de rotation est de 100rpm (tours par minute). Le volume du bol de dissolution est de 1L et le milieu de dissolution utilisé est de l'eau purifiée à pH 7 ou une solution tamponnée à pH 1,3.

Le sel précurseur du cycle de Krebs, et notamment le sel de citrate, est dosé comme il est connu de l'homme du métier. Par exemple, le citrate de potassium libéré est dosé avec un photomètre de flamme, la technique analytique ayant été validée selon les recommandations ICH CPMP/ICH/381/95 - ICH Q2 (R1).

De préférence, le sel précurseur du cycle de Krebs est totalement dissous (taux de dissolution de 100%) en environ quatre heures, que le pH soit de 7 ou de 1,3.

La première formulation selon l'invention comprend le plus souvent de 40% à 80%, de préférence de 50 à 70%, en poids de sel précurseur du cycle de Krebs sur la base du poids total de la première formulation.

Le sel précurseur du cycle de Krebs est ainsi présent à dose physiologiquement efficace ou représentant un multiple ou un sous-multiple d'une dose efficace pour un patient type.

L'agent d'enrobage du micro-comprimé de première formulation est généralement choisi parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, la gomme de xanthane et l'oxyde de polyéthylène, les cires de type cire de paraffine, cire d'abeille ou cire de Carnauba, les copolymères d'ammonium méthacrylate de type A et B tels que décrits dans la Pharmacopée Européenne, et les polyacrylates de dispersion environ 30% tels que décrits dans la Pharmacopée Européenne. L'agent d'enrobage est de façon préférée selon l'invention un polymère d'éthylcellulose.

Selon un mode de réalisation de l'invention, l'enrobage comporte, outre un agent d'enrobage tel que choisi dans la liste ci-dessus, un agent aromatisant et/ou un colorant.

L'épaisseur et l'homogénéité de l'enrobage est un paramètre important de l'invention, car il influence la diffusion du sel précurseur du cycle de Krebs au travers de l'enrobage et donc la cinétique de dissolution de ce sel. Le choix de la nature et de la quantité de l'agent d'enrobage utilisé est aussi un paramètre important de l'invention.

La première formulation pharmaceutique selon l'invention comprend généralement de 0,01% à 5%, de préférence de 0,01% à 2% en poids, de façon encore plus préférée de 1,4 à 2,5%, d'agent d'enrobage du micro-comprimé de première formulation par rapport au poids total de la première formulation.

La première formulation pharmaceutique selon l'invention peut comprendre en outre :
- de 10% à 40%, de préférence de 25% à 35% en poids, par rapport au poids total de la première formulation, d'un liant choisi parmi les celluloses microcristallines, la polyvidone, la polyvinylpyrrolidone, la copovidone, la gomme laque (shellac), la gélatine, les polyméthacrylates, les résines synthétiques, les acrylates, la maltodextrine, et les amidons, et de préférence le liant comporte au moins une cellulose microcristalline ;
- de 0,01% à 5%, de préférence de 0,02% à 3% en poids, par rapport au poids total de la première formulation, d'un agent d'écoulement (ou lubrifiant) choisi parmi l'acide stéarique, le polyéthylène glycol, le stéarate de magnésium, le stéarate de calcium, le stéarate de zinc, le talc, le dioxyde de silice, l'huile de castor hydrogénée, le glycéryl béhénate, et le glycéryl palmitostéarate, et de préférence l'agent d'écoulement est choisi parmi le stéarate de magnésium et le glycéryl béhénate ; et/ou
- tout excipient pharmaceutique approprié, en une quantité classiquement utilisée dans le domaine considéré, par exemple de 0,0001% à 20% du poids total de la première formulation.

L'excipient pharmaceutique de première formulation est généralement inerte, c'est-à-dire inactif et non toxique, et acceptable d'un point de vue pharmaceutique. Un tel excipient est le plus souvent choisi parmi les diluants, liants, désagrégeants, agents d'écoulement, lubrifiants, colorants autorisés par l'Administration compétente, dispersants, solubilisants, stabilisants, conservateurs, plastifiants et agents aromatisants. Un tel excipient peut être aussi un support par exemple choisi dans le groupe formé par les celluloses telles que l'hydroxyméthylcellulose, la carboxyméthylcellulose, les cyclodextrines, le polysorbate 80, le mannitol, la gélatine, le lactose, les huiles végétales, les huiles animales, les carbonates, les amidons et l'acacia.

En outre la première formulation selon l'invention peut comprendre au moins un agent matriciel, présent dans le coeur du micro-comprimé, généralement en tant que matrice à libération prolongée, de préférence à une teneur comprise dans une fourchette de 10% à 30%, de façon encore plus préférée de 15% à 25%, en poids par rapport au poids total de la composition. Un tel agent matriciel est de préférence choisi parmi les agents d'enrobage cités précédemment.

La Pharmacopée Européenne (Ph. Eur.) définit, parmi les comprimés à libération modifiée, les comprimés à libération prolongée, les comprimés à libération retardée et les comprimés à libération séquentielle. Les comprimés à libération modifiée sont des comprimés, enrobés ou non, qui sont préparés avec des excipients spéciaux, ou par des procédés particuliers, ou les deux, visant à modifier, la vitesse, le lieu ou le moment de libération de la ou des substances actives.

En général, les comprimés à libération prolongée sont les comprimés permettant de libérer une substance active de façon prolongée dans le temps et selon une cinétique déterminée. Cela est de préférence effectué en réalisant un coeur de comprimé, ou un comprimé nu (i.e. sans enrobage) utilisant une matrice à libération prolongée dans laquelle est présente la ou les substances actives. Une matrice à libération prolongée est en général un système matriciel, le plus souvent un réseau polymérique, qu'il soit hydrophile ou lipophile. La diffusion de la ou les substances actives au sein de ce réseau est généralement influencée non seulement par les propriétés physico-chimiques inhérentes à cette ou ces substances actives (telle que solubilité, poids moléculaire...), mais également par celles caractérisant le réseau matriciel (tels que: hydrophilie, degré de polymérisation, vitesse de gélification, érosion).

La Pharmacopée Européenne (Ph. Eur.) définit un comprimé comme une préparation solide contenant une unité de prise d'une ou plusieurs substances actives. Les comprimés sont obtenus en agglomérant par compression un volume constant de particules, ou par un autre procédé de fabrication approprié tel que l'extrusion, le moulage ou la cryodessication (lyophilisation). Les comprimés sont destinés à la voie orale. Les comprimés se présentent généralement sous la forme d'un cylindre droit dont les faces inférieures et supérieures peuvent être plates ou convexes et les bords biseautés. La taille d'un comprimé, ou dimension moyenne, est donc généralement le diamètre de ce cylindre, ou un équivalent. Par contre si la hauteur du cylindre est importante, et supérieure au diamètre du cylindre, la taille du comprimé est la hauteur de ce cylindre.

Par "micro-comprimé", on entend selon l'invention un comprimé de taille comprise dans une fourchette de 2 à 4 mm (en général avec une précision sur la taille de ±10%). De préférence, tous les micro-comprimés de la première formulation ont sensiblement la même composition et présentent un taux de dissolution similaire, qui est le taux de dissolution pouvant caractériser la première formulation pharmaceutique de l'invention. Ce taux de dissolution est couramment établi sur la base d'une unité de la préparation, soit dans le cadre de l'invention d'un gramme de micro-comprimés.

Les micro-comprimés de première formulation selon l'invention sont enrobés, ce qui permet un masquage du goût.

Selon un mode de réalisation de l'invention, la première formulation comprend de 55% à 70% de citrate de potassium, de 20 à 30% de cellulose microcristalline, de 0,02% à 2% de stéarate de magnésium, de 0,01% à 1% de glycéryl béhénate et de 1 à 3% d'éthyl cellulose, par rapport au poids total de la première formulation.

### Seconde formulation pharmaceutique

Le sel de bicarbonate est de préférence choisi parmi le bicarbonate de potassium, le bicarbonate de sodium et le bicarbonate de magnésium, et de façon encore plus préférée le sel de bicarbonate est le bicarbonate de potassium.

La seconde formulation pharmaceutique permet avantageusement un passage du sel de bicarbonate dans le tractus intestinal de façon contrôlée et prolongée sur au moins 2 heures, de préférence sur au moins 6 à 8 heures, de façon encore plus préférée sur 8 heures.

La seconde formulation pharmaceutique selon l'invention permet très avantageusement une libération continue *in vivo* de sel de bicarbonate après la prise d'une dose unique, i.e. une prise unique, sur une durée longue, généralement après un quart d'heure et jusqu'à douze heures, de façon prolongée. La libération commence généralement peu après cette prise unique, soit le plus souvent à partir d'un quart d'heure après cette prise, même si la libération peut commencer dès la prise. Par libération continue, on entend selon l'invention une libération qui s'effectue constamment *in vivo,* depuis la prise de la composition jusqu'à une durée d'environ douze heures. La cinétique de cette libération est généralement proche d'une cinétique d'ordre zéro. Une telle libération est qualifiée de « prolongée » car elle atteint ou dépasse une durée d'une heure.

De préférence, la seconde formulation pharmaceutique selon l'invention est telle qu'elle libère *in vivo* une majorité du sel de bicarbonate (c'est-à-dire au moins 50% dudit sel) sur une durée comprise entre huit et douze heures après une prise unique de la composition.

Sans vouloir être liée par une quelconque hypothèse, la demanderesse pense que le mécanisme d'action est tel que, lorsque la seconde formulation est administrée par voie orale à un sujet, le principe actif se libère de façon contrôlée et prolongée : le sel de bicarbonate est absorbé tout au long du tractus digestif.

Avantageusement, la tolérance gastrique de la seconde formulation est améliorée par rapport aux compositions connues de l'art antérieur. En effet, la libération du sel de bicarbonate ayant généralement lieu sur plus de huit heures, il n'y a pas d'intolérance au potassium ni d'alcalose lors de la prise de la dose. Il n'y a donc pas d'effets secondaires associés à une alcalose métabolique ou à des troubles digestifs, tels que les diarrhées.

Selon une variante préférée, la seconde formulation selon l'invention est apte à libérer (ou dissoudre) le sel de bicarbonate *in vitro* dans un milieu de dissolution d'eau purifiée à pH 7 avec un appareil de dissolution de type 2, conforme à la Pharmacopée Européenne (Ph. Eur.) 2.9.3 « *Essai de dissolution des formes solides* », à un taux d'au plus 50% en 4 heures, au plus 75% en 6 heures, et au plus 90% en 8 heures.

De façon particulièrement préférée selon l'invention, indépendamment ou non de la variante précédente, la seconde formulation selon l'invention est apte à libérer (ou dissoudre) le sel de bicarbonate *in vitro* dans un milieu de dissolution d'eau purifiée à pH 7 avec un appareil de dissolution de type 2, conforme à la Pharmacopée Européenne (Ph. Eur.) 2.9.3 « *Essai de dissolution des formes solides* », à un taux compris dans une fourchette de 5% à 15% en une heure, à un taux compris dans une fourchette de 35% à 55% en cinq heures, et à un taux compris dans une fourchette de 70% à 90% en dix heures.

Ce pH de 7 est une mesure aisée à pratiquer en laboratoire, car c'est le pH de l'eau purifiée. La mesure se fait donc simplement par dissolution dans de l'eau purifiée.

Selon une variante préférée, la seconde formulation selon l'invention est apte à libérer (ou dissoudre) le sel de bicarbonate *in vitro* dans un milieu de dissolution de solution tamponnée à pH 1,3 avec un appareil de dissolution de type 2, conforme à la Pharmacopée Européenne (Ph. Eur.) 2.9.3 « *Essai de dissolution des formes solides* », à un taux d'au plus 50% en 4 heures, au plus 75% en 6 heures, et au plus 90% en 8 heures.

De façon particulièrement préférée selon l'invention, indépendamment ou non de la variante précédente, la seconde formulation selon l'invention est apte à libérer (ou dissoudre) le sel de bicarbonate *in vitro* dans un milieu de dissolution de solution tamponnée à pH 1,3 avec un appareil de dissolution de type 2, conforme à la Pharmacopée Européenne (Ph. Eur.) 2.9.3 « *Essai de dissolution des formes solides* », à un taux compris dans une fourchette de 5% à 15% en une heure, à un taux compris dans une fourchette de 35% à 55% en cinq heures, et à un taux compris dans une fourchette de 70% à 90% en dix heures.

De façon générale, la dissolution de la seconde formulation selon l'invention *in vitro* dans un milieu de dissolution donné, selon les conditions décrites plus haut, se produit à pH indépendant. Cela veut dire que, quelque soit le pH du milieu de dissolution dans une plage entre 1,3 et 7, la dissolution se produit selon la même cinétique. La demanderesse a choisi ici deux milieux de dissolution différents, chacun caractérisé par son pH, à savoir pH 1,3 et pH 7, pour définir ce profil de façon caractéristique, selon un test facilement reproductible *in vitro.*

Le test de dissolution du sel de bicarbonate se pratique dans les mêmes conditions que le test de dissolution du sel précurseur de cycle de Krebs.

Le sel de bicarbonate est dosé comme il est connu de l'homme du métier. Par exemple, le bicarbonate de potassium libéré est dosé par conductimétrie, la technique analytique ayant été validée selon les recommandations ICH CPMP/ICH/381/95 - ICH Q2 (R1).

Au sein de la composition selon l'invention, le sel de bicarbonate ne commence de préférence sa dissolution qu'après un quart d'heure (taux de dissolution généralement proche d'environ 0%), puis la cinétique de dissolution est quasiment d'ordre zéro.

La seconde formulation selon l'invention comprend le plus souvent de 40% à 80%, de préférence de 50 à 80%, par exemple de 50 à 70%, en poids de sel de bicarbonate sur la base du poids total de la seconde formulation.

L'agent d'enrobage du mini-comprimé de seconde formulation est généralement choisi parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, la gomme de xanthane et l'oxyde de polyéthylène les cires de type cire de paraffine, cire d'abeille ou cire de Carnauba, les copolymères d'ammonium méthacrylate de type A et B tels que décrits dans la Pharmacopée Européenne, et les polyacrylates de dispersion environ 30% tels que décrits dans la Pharmacopée Européenne. L'agent d'enrobage est de façon préférée selon l'invention un polymère d'éthylcellulose.

Selon l'invention, l'enrobage comporte de préférence, outre un agent d'enrobage tel que choisi dans la liste ci-dessus, un agent aromatisant, et un colorant.

L'épaisseur et l'homogénéité de l'enrobage est un des paramètres essentiels de l'invention, car il influence la diffusion du sel de bicarbonate au travers de l'enrobage et donc la cinétique de dissolution de ce sel. Le choix de la nature et de la quantité de l'agent d'enrobage utilisé est aussi un paramètre important de l'invention.

La seconde formulation pharmaceutique selon l'invention comprend généralement de 1% à 20%, de préférence de 1,5% à 3% en poids d'agent d'enrobage du mini-comprimé de seconde formulation, par rapport au poids total de la seconde formulation.

La matrice à libération prolongée de la seconde formulation est une matrice hydrophile, c'est-à-dire formée d'un matériau susceptible de se gélifier et d'absorber un milieu aqueux, i.e. une matrice comprenant des excipients appartenant essentiellement à la classe des polymères thermoplastiques ; ces polymères sont généralement inertes vis-à-vis des tissus biologiques, des autres excipients dans la formulation et de la substance active, ils sont insolubles et non-digestibles dans les fluides du tractus gastro-intestinal. De manière plus préférée, une telle matrice à libération prolongée de la seconde formulation est choisie parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, et les polyacrylates de dispersion environ 30% tels que décrits dans la Pharmacopée Européenne, et de façon encore plus préférée cette matrice à libération prolongée est une hydroxypropylméthyl cellulose.

La seconde formulation pharmaceutique selon l'invention comprend généralement de 10% à 30%, de préférence de 15 à 25% en poids de matrice hydrophile à libération prolongée du mini-comprimé de seconde formulation, par rapport au poids total de la seconde formulation.

La seconde formulation pharmaceutique selon l'invention peut comprendre en outre :
- de 5% à 20%, de préférence de 5% à 10% en poids, par rapport au poids total de la seconde formulation, d'un liant choisi parmi les celluloses microcristallines, la polyvidone, la polyvinylpyrrolidone, la copovidone, la gomme laque (shellac), la gélatine, les polyméthacrylates, les résines synthétiques, les acrylates, la maltodextrine, et les amidons, et de préférence le liant comporte au moins une cellulose microcristalline ;
- de 0,01% à 5%, de préférence de 0,01% à 3% en poids, par rapport au poids total de la seconde formulation, d'un agent d'écoulement choisi parmi l'acide stéarique, le polyéthylène glycol, le stéarate de magnésium, le stéarate de calcium, le stéarate de zinc, le talc, le dioxyde de silice, l'huile de castor hydrogénée, le glycéryl béhénate, et le glyceryl palmitostéarate, et de préférence l'agent d'écoulement est le stéarate de magnésium ; et/ou
- tout excipient pharmaceutique approprié, en une quantité classiquement utilisée dans le domaine considéré, par exemple de 0,0001% à 20% du poids total de la seconde formulation.

L'excipient pharmaceutique de seconde formulation est choisi, indépendamment de l'excipient pharmaceutique de la première formulation, de la même façon que l'excipient de première formulation.

De préférence, tous les mini-comprimés ont la même composition et présentent un taux de dissolution similaire, qui est le taux de dissolution pouvant caractériser la seconde formulation pharmaceutique de l'invention.

La seconde formulation pharmaceutique se présente sous la forme de mini-comprimés.

Par "mini-comprimé", on entend selon l'invention un comprimé de taille d'au moins 2 mm, par exemple comprise dans une fourchette de 2 à 25 mm (en général avec une précision sur la taille de ±10%). L'homme du métier est à même de choisir la taille de comprimé. Ces comprimés peuvent être des micro-comprimés, ou bien des comprimés de taille plus élevée, par exemple comprise dans une fourchette de 4 à 25 mm. Selon un mode de réalisation préféré de l'invention, le mini-comprimé de seconde formulation est de préférence un "micro-comprimé".

De préférence, tous les mini-comprimés de la seconde formulation ont la même composition et présentent un taux de dissolution similaire, qui est le taux de dissolution pouvant caractériser la seconde formulation pharmaceutique de l'invention. Ce taux de dissolution est couramment établi sur la base d'une unité de la préparation, soit dans le cadre de l'invention d'un gramme de micro-comprimés.

Les mini-comprimés de seconde formulation selon l'invention sont enrobés, ce qui permet un masquage du goût.

Selon un mode de réalisation de l'invention, la seconde formulation comprend de 60% à 70% de bicarbonate de potassium, de 15 à 25% d'hypromellose, de 7 à 17% de cellulose microcristalline, de 1 à 3% de glycéryl béhénate, de 0,01 % à 1 % de stéarate de magnésium, et de 1,5 à 3% d'éthyl cellulose, par rapport au poids total de la seconde formulation.

L'hypromellose est une hydroxypropylméthyl cellulose.

### Composition selon l'invention

La composition selon l'invention combine avantageusement les modes de réalisation préférés de la première formulation pharmaceutique, tels que décrits précédemment, et les modes de réalisation préférés de la seconde formulation pharmaceutique, tels que décrits précédemment. A cet égard, toutes les combinaisons possibles sont envisagées dans le cadre de l'invention, comme exprimé dans les revendications. Par exemple, la composition selon l'invention est de préférence telle que le sel précurseur du cycle de Krebs est un sel de citrate, de façon encore plus préférée du citrate de potassium, et telle que le sel de bicarbonate est du bicarbonate de potassium.

Selon l'invention, le patient ingère généralement plusieurs comprimés à chaque prise, selon la dose thérapeutique qui lui est appropriée (dose journalière divisée par le nombre de prises par jour).

Dans tous les cas, une prise de médicament correspond à plusieurs micro-comprimés et plusieurs mini-comprimés, c'est-à-dire à un ensemble de micro-comprimés et de mini-comprimés.

L'invention vise donc à couvrir également un ensemble de micro-comprimés et de mini-comprimés, correspondant à une prise thérapeutique. L'homme du métier est à même d'évaluer le nombre de micro-comprimés et de mini-comprimés correspondant à une dose thérapeutique, en fonction des besoins de la personne, de son âge, de son poids, en fonction de la quantité de sel précurseur du cycle de Krebs par micro-comprimé et de sel de bicarbonate par mini-comprimé, ainsi que le nombre de prises par jour.

La composition pharmaceutique selon l'invention se présente sous la forme de micro-comprimés de première formulation, et de mini-comprimés de seconde formulation, i.e. un ensemble de comprimés de deux types différents.

Cependant, un mode de réalisation préféré selon l'invention est quand les comprimés de seconde formulation sont des micro-comprimés, i.e. que les comprimés de la composition sont tous des micro-comprimés. Cela est vrai que la taille des micro-comprimés de première formulation soit identique ou différente de la taille des micro-comprimés de seconde formulation. Bien entendu, le cas préféré est le cas où la taille des micro-comprimés de première formulation est identique à la taille des micro-comprimés de seconde formulation. Dans tous les cas, les principes actifs que sont le sel précurseur du cycle de Krebs et le sel de bicarbonate sont présents dans la composition à dose physiologiquement efficace ou représentant un multiple ou un sous-multiple d'une dose efficace pour un patient type.

Ceci représente des taux de principes actifs, en poids par rapport au poids total de la composition, importants par rapport à ce qui est connu. Cela permet avantageusement de minimiser le volume de la composition pharmaceutique, et donc le volume de prise journalière. Par voie de conséquence, on obtient ainsi une meilleure acceptation par le patient.

Ceci est particulièrement appréciable pour les prises de composition en dose élevée et/ou pour les traitements thérapeutiques pédiatriques.

Du fait de la petite taille du micro-comprimé, un seul micro-comprimé de chacune des deux formulations n'est généralement pas suffisant pour une prise, et à chaque prise, plusieurs micro-comprimés de chacune des deux formulations sont administrés. Un des avantages de la forme en plusieurs micro-comprimés est que la prise par le patient est facilitée, par rapport à une prise d'un seul comprimé de volume plus important. Ceci est particulièrement avantageux lorsque le patient est un enfant.

La composition selon la présente invention peut être utilisée chez le mammifère, plus précisément chez l'homme, et tout particulièrement chez l'enfant.

La composition selon l'invention a la particularité de libérer de façon continue et séquentielle le sel précurseur du cycle de Krebs et le sel de bicarbonate tout au long du tractus digestif. Ainsi, dans un premier temps, le sel précurseur du cycle de Krebs est libéré dans l'estomac, absorbé principalement au niveau du duodénum, et excrété dans les urines, où, lorsqu'il est un sel de citrate, il se complexe en partie avec le calcium dans les urines, évitant la formation de calculs de phosphate de calcium et d'oxalate de calcium. Le sel de bicarbonate prend le relai et prolonge l'effet alcalinisant car il est absorbé majoritairement dans tout le reste du tractus digestif jéjunum, iléon et colon, et excrété dans les urines. Il en résulte au fil du temps, une excrétion lente et continue des principes actifs alcalinisants dans les urines, ce qui provoque un maintien du pH urinaire à une valeur supérieure à 7 pendant au moins 8 heures, puisque la totalité des sels est libérée durant au moins 8 heures après ingestion. Cette composition permet donc une couverture de l'alcalinisation du patient sur une nuit complète avec une seule dose (correspondant à une prise unique) avant le coucher.

La libération prolongée observée *in vitro* pour chacune des deux formulations reflète une libération contrôlée dans l'organisme, qui peut être vérifiée par mesure du pH urinaire des patients traités par cette composition.

La composition que la Demanderesse a mise au point présente l'avantage que l'on observe, de façon surprenante, non pas un antagonisme entre les deux sels présents dans la composition, mais une synergie. Cela est dû à la libération séquentielle et optimisée de ces deux sels dont les deux mécanismes d'action deviennent complémentaires. C'est un réel progrès par rapport aux compositions de l'art antérieur.

La tolérance gastrique est améliorée avec la composition selon l'invention, par rapport aux formulations de l'art antérieur, et on évite l'alcalose sanguine trop brutale, puisque la libération alcaline est lente permettant aux mécanismes physiologiques de réguler sans à-coup le pH sanguin et sans entraîner des phénomènes de crampes gastriques douloureuses.

La composition selon l'invention est particulièrement bien adaptée à la prévention et au traitement de la cystinurie, car elle permet un maintien du pH urinaire aux valeurs préconisées, de façon plus efficace que les formulations de l'art antérieur. Elle permet de réduire la survenue de lithiase et les complications sur le long terme, et de diminuer les interventions chirurgicales.

La composition selon l'invention est donc utilisable comme médicament, en particulier pour le traitement et/ou la prévention de la cystinurie. Cela inclut la prévention et /ou le traitement des complications associées à la cystinurie.

Les micro-comprimés et mini-comprimés selon l'invention sont particulièrement bien adaptés au traitement et/ou à la prévention de la cystinurie, du fait de leur profil de libération optimale.

Le procédé de fabrication comprend 4 étapes :
La première étape est une étape de mélange du principe actif, de préférence du seul principe actif, avec les autres ingrédients constituant le coeur de comprimé, séparément pour chacune des formulations. Chaque mélange est réalisé dans un mélangeur par gravité de type Stuart STR4, mais peut être réalisé dans tout autre type de mélangeur industriel.
La deuxième étape est une étape de fabrication des deux types de comprimés, à partir des deux mélanges issus de la première étape, séparément pour chacune des formulations. Cette seconde étape est généralement réalisée par une première opération de compression directe dans une presse rotative, par exemple pour la fabrication de micro-comprimés de dimension 2 mm (de type PR12) à l'aide de six supports possédant chacun une tête à six poinçons de 2mm. Cette deuxième étape comprend ensuite le plus souvent une seconde opération de dépoussiérage des comprimés fabriqués au cours de la première opération.
La troisième étape est une étape d'enrobage, par l'agent d'enrobage, des comprimés issus de la deuxième étape, séparément pour chacune des formulations. L'agent d'enrobage est généralement appliqué sous forme de solution ou de suspension dans des conditions qui favorisent l'évaporation du solvant.
La quatrième étape est une étape de remplissage homogène des contenants pouvant être des flacons, des sachets, des gélules, des ampoules etc. ...), par chacune des deux formulations. Cette étape est effectuée en fonction du pourcentage respectif de chaque formulation afin d'obtenir le ratio formulation 1/formulation 2 choisi par exemple : 33% de micro-comprimés de la formulation 1 et 67% de mini-comprimés de la formulation 2.

L'invention est illustrée par les figures 1 à 4 ci-jointes, parmi lesquelles :
- la figure 1 représente le profil de dissolution en taux T de dissolution (pourcentage de principe actif -citrate de potassium) en fonction de la durée D (h:min) pour une première formulation identifiée par A ;
- la figure 2 représente le profil de dissolution en taux T de dissolution (pourcentage de principe actif -bicarbonate de potassium) en fonction de la durée D (h:min) pour une composition identifiée par I ;
- la figure 3 représente les variations du pH urinaire en fonction des heures (h-h) sur une journée pour un sujet ; et
- la figure 4 représente les variations du pH urinaire en fonction des heures (h-h) sur une journée pour trois autres sujets.

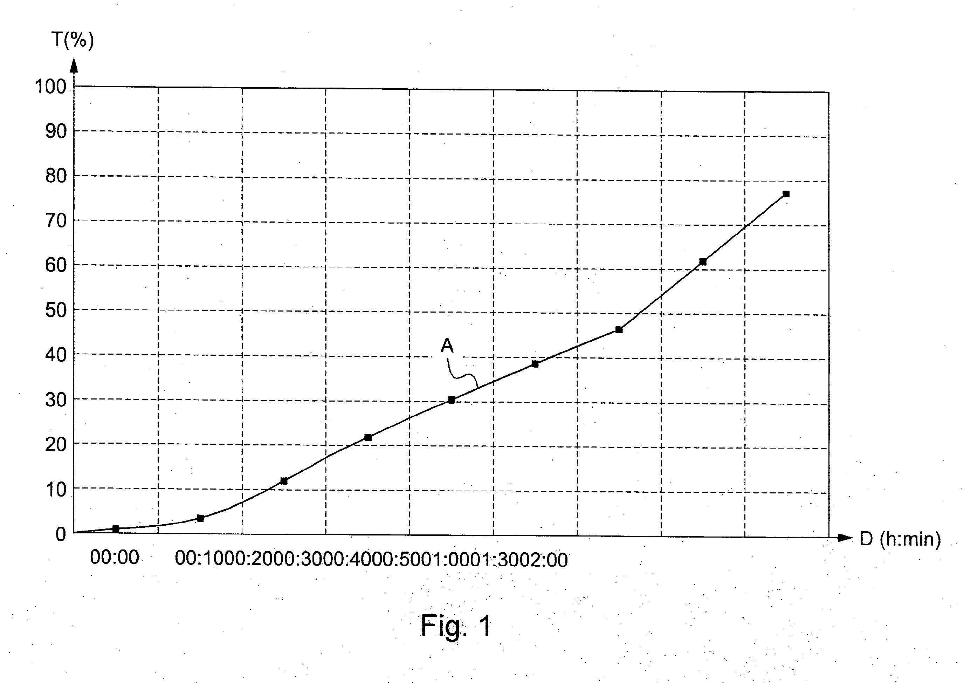

Les figures 1, 2, 3 et 4 sont commentées respectivement dans les exemples 1, 2, 3 et 4 ci-après.

Les exemples suivants illustrent l'invention sans pour autant la limiter.

### Exemple 1 :

Un lot de micro-comprimés de taille (diamètre moyen) 2mm est réalisé selon le procédé décrit précédemment à savoir, une étape de mélange des poudres, suivie d'une étape de compression, puis d'une étape d'enrobage. Ce lot est le lot A, constitué de 200g de micro-comprimés. Ces micro-comprimés ont la composition suivante :
Citrate de potassium (principe actif, source Dr Paul Lohmann) : 66,9%
Cellulose microcristalline (liant, Ceolus® KG-802 de la société Asahi) : 19,7%
Cellulose microcristalline (liant, Ceolus® UF-711 de la société Asahi) : 9,8%
Stéarate de magnésium (agent d'écoulement) : 2,0%
Glyceryl béhénate (agent lubrifiant, référence commerciale Compritol® ATO 888 de la société GATTEFOSSE) : 0,01% ;
Polymère d'éthyl cellulose (agent d'enrobage, référence commerciale Ethocel® 20 standard premium de la société Dow) : 1,66%.

Ces micro-comprimés sont très bien acceptés et tolérés par les patients. En outre, ils sont sans goût et faciles à avaler.

La figure 1 montre le profil de dissolution in vitro d'un gramme de ces micro-comprimés dans l'eau, dans les conditions décrites ci-après, sur une durée de 2 heures. Les micro-comprimés A ont été placés dans un banc de dissolution Pharmatest modèle PTW S3C, dans lesquelles les conditions de températures sont 37°C±0.5°C, et la vitesse de rotation est de 100rpm. Le milieu de dissolution est de l'eau purifiée à pH 7. On a obtenu une courbe A qui illustre une libération de citrate de potassium qui se fait de façon progressive et régulière, Comme illustré sur la Figure 1, les micro-comprimés sont aptes à libérer le sel de citrate *in vitro* dans un milieu de dissolution d'eau purifiée à pH 7 à un taux de 4,5% en 15 minutes, de 20,6% en 30 minutes, et de 48,6% en une heure.

### Exemple 2 :

Un lot I de micro-comprimés de taille (diamètre moyen) 2mm a été réalisé selon le procédé décrit précédemment, à savoir une étape de mélange des poudres, suivie d'une étape de compression puis d'une étape d'enrobage, à raison de 200g de micro-comprimés par lot. Ces comprimés ont la composition suivante:
Bicarbonate de potassium (principe actif, source Dr Paul Lohmann) : 66,4%
Hypromellose (matrice, HPMC 100 000 90SH) : 19,5%
Cellulose microcristalline (liant, référence commerciale Ceolus® UF-711 de la société Asahi-Kasei) : 9,8%
Stéarate de magnésium (agent d'écoulement) : 0,01%
Glyceryl béhénate (agent lubrifiant, référence commerciale Compritol® ATO 888 de la société GATTEFOSSE) : 2%
Ethyl cellulose (polymère) (matériau d'enrobage, référence commerciale Ethocel® 20 standard premium de la société Dow Chemical) : 2,3%.

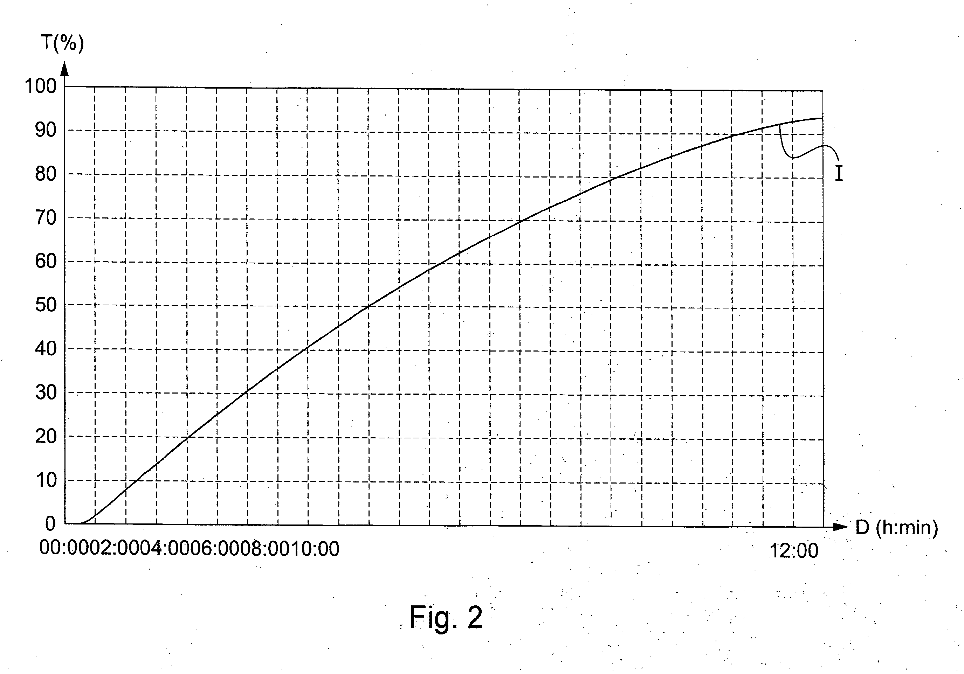

La courbe I de la figure 2 montre le profil de dissolution in vitro de tels micro-comprimés dans l'eau purifiée à pH 7.

Un tel profil a été réalisé en plaçant les mini-comprimés dans un banc de dissolution Pharmatest modèle PTW S3C, à température 37°C±0,5°, à volume du bol de dissolution de 1 L et à vitesse de rotation est de 100rpm.

Le dosage du bicarbonate de potassium est réalisé par conductimétrie selon une méthode analytique validée selon les recommandations ICH CPMP/ICH/381/95 - ICH Q2 (R1).

Les micro-comprimés I sont très bien acceptés et tolérés par les patients. En outre, ils sont sans goût et faciles à avaler.

La courbe I de la figure 2 illustre une libération de bicarbonate de potassium qui se fait de façon progressive et régulière, répondant aux critères d'un taux d'au plus 50% en 4 heures, au plus 75% en 6 heures, et au plus 90% en 8 heures.

En outre, la courbe I illustre une libération de bicarbonate de potassium qui aboutit à une dissolution quasiment complète au bout de 12 à 15 heures.

### Exemple 3 :

Une composition selon l'invention a été réalisée, sur la base d'un tiers (en % poids) de première formulation de l'exemple 1 et de deux tiers (en % poids) de seconde formulation de l'exemple 2.

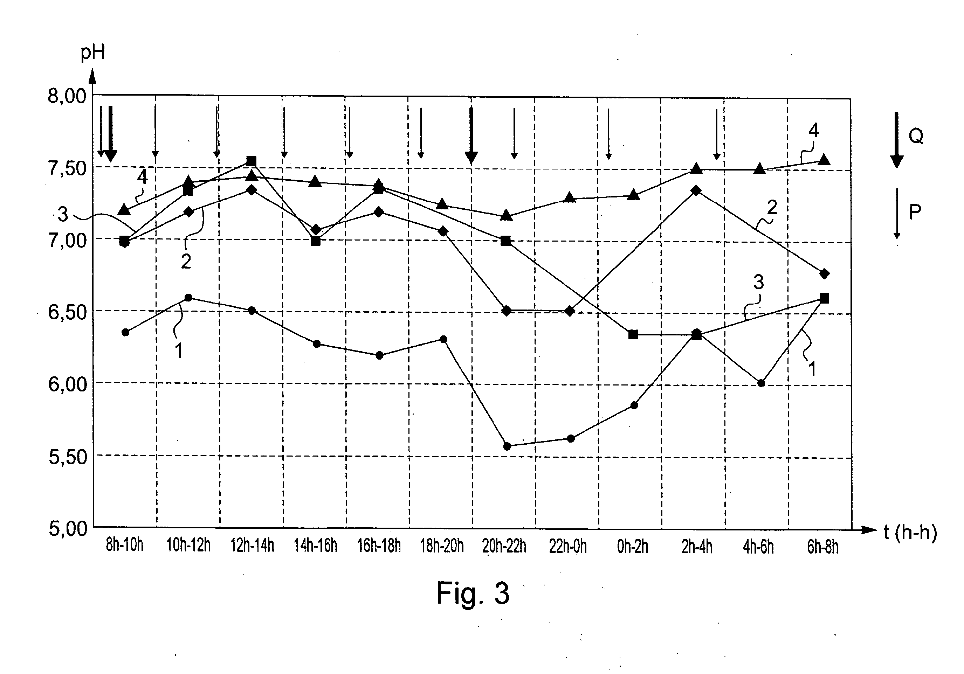

Un sujet sain présente une ligne de base de pH urinaire représentée sur la figure 3 par la courbe 1, sur une journée sans traitement.

Le sujet était sous régime alimentaire sans boisson alcoolisée, sans boisson bicarbonatée, et réduit en aliment contenant de l'acide citrique (i.e. pas de boisson de type jus d'orange ni d'aliment en conserve contenant de l'acide citrique). En outre, les viandes rouges et les fromages étaient proscrits.

Trois essais différents ont été réalisés sur ce sujet (courbes 2, 3 et 4). La mesure du pH urinaire est effectuée sur les urines fraîches dans les secondes qui suivent la miction à l'aide d'un pH mètre à électrode.

La courbe 2 montre les mesures du pH urinaire du patient à intervalle régulier tout au long du nycthémère pour une dose journalière de 18 grammes en neuf prise (représentées chacune par une flèche P),de citrate en préparation officinale à libération immédiate.

La courbe 3 montre les mesures du pH urinaire du patient à intervalles réguliers tout au long du nycthémère pour une dose journalière de 18 grammes en neuf prises (représentées chacune par une flèche P), de bicarbonate de potassium en préparation officinale à libération immédiate.

La courbe 4 montre les mesures du pH urinaire du patient à intervalles réguliers tout au long du nycthémère pour une dose journalière de 18 grammes en seulement deux prises (représentées chacune par une flèche Q), de composition selon l'invention.

Il est apparent sur la figure 3 que seule la composition selon l'invention permet le maintien du pH urinaire du patient à des valeurs comprises entre 7 et 7,6, tout au long de la journée. Cela est d'autant plus remarquable que seulement deux prises journalières permettent d'arriver à un tel résultat.

### Exemple 4 :

La composition de l'exemple 3 a fait l'objet d'essais, sur trois sujets sains différents (deux hommes référencés 1 et 3, et une femme référencée 2).

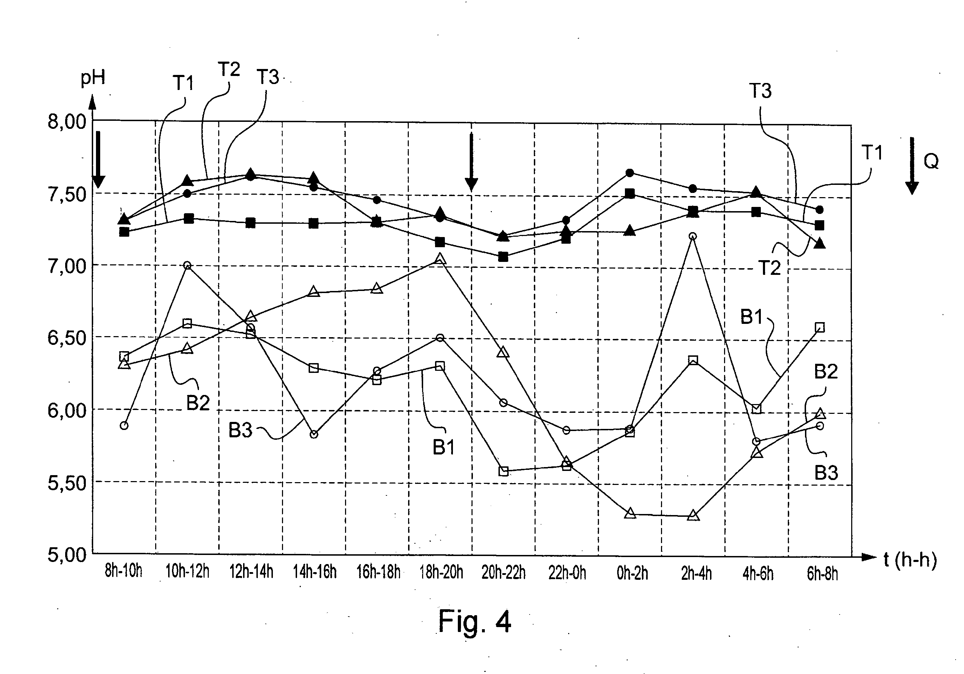

La figure 4 représente les variations du pH urinaire en fonction des heures sur une journée.

Pour chacun de ces patients i (i = 1, 2 ou 3), une courbe Bi de ligne de base est réalisée, de pH urinaire sur une journée sans traitement.

Ensuite il a été administré à ces sujets sains deux doses par jour de la composition selon l'invention, une dose de 9g pour chacun des deux sujets masculins et de 6g pour le sujet féminin. La mesure du pH urinaire est effectuée sur les urines fraîches dans les secondes qui suivent la miction à l'aide d'un pH mètre à électrode. Les sujets étaient tous sous le même régime alimentaire que le sujet décrit à l'exemple 3.

Les courbes T1 et T2 présentées représentent pour les sujets 1 et 2 la moyenne de deux expérimentations conduites à un mois d'intervalle dans les mêmes conditions.

Par mesure du pH urinaire lors de la prise de la composition selon l'invention (courbes T1, T2, et T3), par rapport à une référence sans prise de médicament (courbes B1, B2 et B3), on voit que la composition de l'invention permet d'obtenir une régulation du pH urinaire satisfaisante, en ce que ce pH n'est jamais au-dessous de la valeur de 7.

## Revendications

1. Composition pharmaceutique solide à usage oral sous forme de comprimés comprenant :
- une première formulation pharmaceutique solide à usage oral sous forme d'au moins un micro-comprimé, le micro-comprimé ayant une taille comprise dans une fourchette de 2 à 4 mm, et étant constitué d'un coeur comprenant au moins un sel précurseur du cycle de Krebs en tant que principe actif, et d'un enrobage comprenant au moins un agent d'enrobage, le sel précurseur du cycle de Krebs étant choisi parmi les fumarates, les malates, les citrates, l'alpha-cétoglutarate, le succinyl-Coenzyme A, les succinates et l'oxaloacétate,
et
- une seconde formulation pharmaceutique à usage oral sous forme d'au moins un mini-comprimé, le mini-comprimé ayant une taille comprise dans une fourchette de 2 à 25 mm, et le mini-comprimé étant constitué d'un coeur comprenant au moins un sel de bicarbonate en tant que principe actif et au moins une matrice hydrophile à libération prolongée, et d'un enrobage comportant au moins un agent d'enrobage, la dissolution in vitro de la seconde formulation dans un milieu de dissolution tamponné à pH donné dans une plage entre 1,3 et 7, avec un appareil de dissolution de type 2, conforme à la Pharmacopée Européenne (Ph. Eur.) 2.9.3 « *essai de dissolution des formes solides* », se produisant selon une cinétique indépendante du pH.

2. Composition selon la revendication précédente, telle qu'elle comprend de 30 à 70% de première formulation et de 70 à 30% de seconde formulation, en poids par rapport au poids total de la composition.

3. Composition selon l'une quelconque des revendications précédentes, telle que la première formulation comprend de 40% à 80%, de préférence de 50 à 70%, en poids de sel précurseur du cycle de Krebs sur la base du poids total de la première formulation et telle que la seconde formulation comprend de 40% à 80%, de préférence de 50 à 80%, en poids de sel de bicarbonate sur la base du poids total de la seconde formulation.

4. Composition selon l'une quelconque des revendications précédentes, telle que le sel précurseur du cycle de Krebs est un sel de citrate choisi parmi le citrate de potassium, le citrate de sodium et le citrate de magnésium, et de façon préférée le sel de citrate est le citrate de potassium.

5. Composition selon l'une quelconque des revendications précédentes, telle que la première formulation comprend de 0,01% à 5%, de préférence de 0,01% à 2% en poids, de façon encore plus préférée de 1,4 à 2,5%, d'agent d'enrobage du micro-comprimé de première formulation par rapport au poids total de la première formulation.

6. Composition selon l'une quelconque des revendications précédentes, telle que l'agent d'enrobage du micro-comprimé de première formulation est choisi parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, la gomme de xanthane et l'oxyde de polyéthylène, les cires de type cire de paraffine, cire d'abeille ou cire de Carnauba, les copolymères d'ammonium méthacrylate de type A et B tels que décrits dans la Pharmacopée Européenne, et les polyacrylates de dispersion environ 30% tels que décrits dans la Pharmacopée Européenne ; et de façon préférée l'agent d'enrobage est un polymère d'éthylcellulose.

7. Composition selon l'une quelconque des revendications précédentes, telle que le sel de bicarbonate est choisi parmi le bicarbonate de potassium, le bicarbonate de sodium et le bicarbonate de magnésium, et de préférence le sel de bicarbonate est le bicarbonate de potassium.

8. Composition selon l'une quelconque des revendications précédentes, telle que la seconde formulation comprend de 1% à 20%, de préférence de 1,5% à 3% en poids d'agent d'enrobage du mini-comprimé de seconde formulation, par rapport au poids total de la seconde formulation.

9. Composition selon l'une quelconque des revendications précédentes, telle que l'agent d'enrobage du mini-comprimé de seconde formulation est choisi parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, la gomme de xanthane et l'oxyde de polyéthylène les cires de type cire de paraffine, cire d'abeille ou cire de Carnauba, les copolymères d'ammonium méthacrylate de type A et B tels que décrits dans la Pharmacopée Européenne, et les polyacrylates de dispersion environ 30% tels que décrits dans la Pharmacopée Européenne ; et de préférence l'agent d'enrobage est un polymère d'éthylcellulose.

10. Composition selon l'une quelconque des revendications précédentes, telle que la seconde formulation comprend de 10% à 30%, de préférence de 15 à 25% en poids de matrice à libération prolongée du mini-comprimé de seconde formulation, par rapport au poids total de la seconde formulation, ladite matrice à libération prolongée de la seconde formulation étant de préférence choisie parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, et les polyacrylates de dispersion environ 30% tels que décrits dans la Pharmacopée Européenne, et de façon encore plus préféré la matrice à libération prolongée étant une hydroxypropylméthyl cellulose.

11. Composition selon l'une quelconque des revendications précédentes, telle que la seconde formulation est apte à libérer le sel de bicarbonate *in vitro* dans un milieu de dissolution d'eau purifiée à pH 7 avec un appareil de dissolution de type 2, conforme à la Pharmacopée Européenne (Ph. Eur.) 2.9.3 « *Essai de dissolution des formes solides* », à un taux d'au plus 50% en 4 heures, au plus 75% en 6 heures, et au plus 90% en 8 heures.

12. Composition selon l'une quelconque des revendications précédentes, telle que la première formulation comprend de 55% à 70% de citrate de potassium, de 20 à 30% de cellulose microcristalline, de 0,02% à 2% de stéarate de magnésium, de 0,01 % à 1 % de glycéryl béhénate et de 1 à 3% d'éthyl cellulose, par rapport au poids total de la première formulation.

13. Composition selon l'une quelconque des revendications précédentes, telle que la seconde formulation comprend de 60% à 70% de bicarbonate de potassium, de 15 à 25% d'hypromellose, de 7 à 17% de cellulose microcristalline, de 1 à 3% de glycéryl béhénate, de 0,01 % à 1% de stéarate de magnésium, et de 1,5 à 3% d'éthyl cellulose, par rapport au poids total de la seconde formulation.

14. Composition selon l'une quelconque des revendications précédentes pour son utilisation comme médicament.

15. Composition selon l'une quelconque des revendications 1 à 15, pour son utilisation comme médicament pour le traitement et/ou la prévention de la cystinurie.

## Patentansprüche

1. Feste pharmazeutische Zusammensetzung zur oralen Verwendung in Tablettenform, umfassend:
- eine erste feste pharmazeutische Formulierung zur oralen Verwendung in Form von wenigstens einer Mikrotablette, wobei die Mikrotablette eine Größe hat, die in einem Bereich von 2 bis 4 mm liegt, und aus einem Kern, der wenigstens ein Krebszyklusvorläufersalz als Wirkstoff umfasst, und einer Beschichtung besteht, die wenigstens ein Beschichtungsmittel umfasst, wobei das Krebszyklusvorläufersalz aus der Gruppe der Fumarate, Malate, Citrate, Alpha-Ketoglutarat, Succinyl-Coenzym A, Succinaten und Oxalacetat ausgewählt ist,
und
- eine zweite pharmazeutische Formulierung zur oralen Verwendung in Form von wenigstens einer Mikrotablette, wobei die Mikrotablette eine Größe hat, die in einem Bereich von 2 bis 25 mm liegt, und aus einem Kern, der wenigstens ein Bicarbonatsalz als Wirkstoff und wenigstens eine hydrophile Matrix mit verzögerter Freisetzung umfasst, und einer Beschichtung besteht, die wenigstens ein Beschichtungsmittel umfasst, wobei die Auflösung in vitro der zweiten Formulierung in einem gepufferten Auflösungsmedium mit einem in einem Bereich zwischen 1,3 und 7 gegebenem pH-Wert mit einer Auflösungsvorrichtung vom Typ 2 gemäß der Europäischen Pharmakopöe (Ph.Eur.) 2.9.3 "*Auflösungstest für feste Formen*" gemäß einer vom pH-Wert abhängigen Kinetik erfolgt.

2. Zusammensetzung nach dem vorhergehenden Anspruch, wobei die Zusammensetzung 30 bis 70 Gewichtsprozent der ersten Formulierung und 70 bis 30 Gewichtsprozent der zweiten Formulierung bezogen auf das Gesamtgewicht der Zusammensetzung umfasst.

3. Zusammensetzung nach einem der vorhergehenden Ansprüche, wobei die erste Formulierung das Krebszyklusvorläufersalz in einem Anteil von 40 bis 80 Gewichtsprozent, vorzugsweise von 50 bis 70 Gewichtsprozent auf der Grundlage des Gesamtgewichts der ersten Formulierung, und die zweite Formulierung das Bicarbonatsalz in einem Anteil von 40 bis 80 Gewichtsprozent, vorzugsweise von 50 bis 80 Gewichtsprozent auf der Grundlage des Gesamtgewichts der zweiten Formulierung umfasst.

4. Zusammensetzung nach einem der vorhergehenden Ansprüche, wobei das Krebszyklusvorläufersalz ein Citratsalz ist, das aus Kaliumcitrat, Natriumcitrat und Magnesiumcitrat ausgewählt ist, und wobei das Citratsalz vorzugsweise Kaliumcitrat ist.

5. Zusammensetzung nach einem der vorhergehenden Ansprüche, wobei die erste Formulierung das Beschichtungsmittel der Mikrotablette der ersten Formulierung in einem Anteil von 0,01 bis 5 Gewichtsprozent, vorzugsweise 0,01 bis 2 Gewichtsprozent, besonders bevorzugt von 1,4 bis 2,5 Gewichtsprozent bezogen auf das Gesamtgewicht der ersten Formulierung umfasst.

6. Zusammensetzung nach einem der vorhergehenden Ansprüche, wobei das Beschichtungsmittel der Mikrotablette der ersten Formulierung aus den Alginaten, Carboxyvinylpolymeren, Natriumsalzen von Carboxymethylcellulose, den Cellulosederivaten einschließlich der Polymere Hydroxypropylmethylcellulose, Hydroxypropylethylcellulose, Hydroxypropylcellulose, Hydroxyethylcellulose, Methylcellulose, Ethylcellulose, Xanthangummi und Polyethlyenoxid, den Wachsen vom Typ Paraffinwachs, Bienenwachs oder Carnaubawachs, den Copolymeren von Ammoniummethacrylat Typ A und B, wie in der Europäischen Pharmakopöe beschrieben, und den Polyacrylat-Dispersionen von etwa 30%, wie in der Europäischen Pharmakopöe beschrieben, ausgewählt ist; und wobei das Beschichtungsmittel vorzugsweise ein Ethylcellulose-Polymer ist.

7. Zusammensetzung nach einem der vorhergehenden Ansprüche, wobei das Bicarbonatsalz ausgewählt ist aus Kaliumbicarbonat, Natriumbicarbonat und Magnesiumbicarbonat, und vorzugsweise das Bicarbonatsalz Kaliumbicarbonat ist.

8. Zusammensetzung nach einem der vorhergehenden Ansprüche, wobei die zweite Formulierung das Beschichtungsmittel der Mikrotablette der zweiten Formulierung in einem Anteil von 1 bis 20 Gewichtsprozent, vorzugsweise von 1,5 bis 3 Gewichtsprozent bezogen auf das Gesamtgewicht der zweiten Formulierung umfasst.

9. Zusammensetzung nach einem der vorhergehenden Ansprüche, wobei das Beschichtungsmittel der Mikrotablette der zweiten Formulierung aus den Alginaten, Carboxyvinylpolymeren, Natriumsalzen von Carboxymethylcellulose, den Cellulosederivaten einschließlich der Polymere Hydroxypropylmethylcellulose, Hydroxypropylethylcellulose, Hydroxypropylcellulose, Hydroxyethylcellulose, Methylcellulose, Ethylcellulose, Xanthangummi und Polyethlyenoxid, den Wachsen vom Typ Paraffinwachs, Bienenwachs oder Carnaubawachs, den Copolymeren von Ammoniummethacrylat Typ A und B, wie in der Europäischen Pharmakopöe beschrieben, und den Polyacrylat-Dispersionen von etwa 30%, wie in der Europäischen Pharmakopöe beschrieben, ausgewählt ist; und wobei das Beschichtungsmittel vorzugsweise ein Ethylcellulose-Polymer ist.

10. Zusammensetzung nach einem der vorhergehenden Ansprüche, wobei die zweite Formulierung die Matrix mit verlängerter Freisetzung der Minitablette der zweiten Formulierung in einem Anteil von 10 bis 30 Gewichtsprozent, vorzugsweise von 15 bis 25 Gewichtsprozent bezogen auf das Gesamtgewicht der zweiten Formulierung umfasst, wobei die Matrix mit verlängerter Freisetzung der zweiten Formulierung vorzugsweise aus den Alginaten, Carboxyvinylpolymeren, Natriumsalzen von Carboxymethylcellulose, den Cellulosederivaten einschließlich den Polymeren Hydroxypropylmethylcellulose, Hydroxypropylethylcellulose, Hydroxypropyl-cellulose, Hydroxyethylcellulose, Methylcellulose, Ethylcellulose, und den Polyacrylaten-Dispersionen von ca. 30%, wie in der Europäischen Pharmakopöe beschrieben, ausgewählt ist; und wobei die Matrix mit verlängerter Freisetzung besonders bevorzugt eine Hydroxypropylmethylcellulose ist.

11. Zusammensetzung nach einem der vorhergehenden Ansprüche, wobei die zweite Formulierung das Bicarbonatsalz in vitro in einem Auflösungsmedium aus gereinigtem Wasser mit einem pH-Wert von 7 mit einer Auflösungsvorrichtung vom Typ 2 gemäß der Europäischen Pharmakopöe (Ph.Eur.) 2.9.3 "*Auflösungstest für feste Formen*" mit einer Rate von höchstens 50% in 4 Stunden, höchstens 75% in 6 Stunden, und höchstens 90% in 8 Stunden freisetzen kann.

12. Zusammensetzung nach einem der vorhergehenden Ansprüche, wobei die erste Formulierung von 55% bis 70% Kaliumcitrat, von 20% bis 30% mikrokristalline Cellulose, von 0,02% bis 2% Magnesiumstearat, von 0,01% bis 1% Glycerylbehenat und von 1% bis 3% Ethylcellulose bezogen auf das Gesamtgewicht der ersten Formulierung umfasst.

13. Zusammensetzung nach einem der vorhergehenden Ansprüche, wobei die zweite Formulierung von 60% bis 70% Kaliumbicarbonat, von 15% bis 25% Hypromellose, von 7% bis 17% mikrokristalline Cellulose, von 1 % bis 3% Glycerylbehenat, von 0,01% bis 1% Magnesiumstearat und von 1,5% bis 3% Ethylcellulose bezogen auf das Gesamtgewicht der zweiten Formulierung umfasst.

14. Zusammensetzung nach einem der vorhergehenden Ansprüche zur Verwendung als Arzneimittel.

15. Zusammensetzung nach einem der Ansprüche 1 bis 15 zur Verwendung als Arzneimittel für die Behandlung und/oder die Prävention von Zystinurie.

## Claims

1. Solid pharmaceutical composition for oral use in the form of tablets comprising:
- a first solid pharmaceutical formulation for oral use in the form of at least one microtablet, said microtablet having a size comprised within a range from 2 to 4 mm and being constituted by a core comprising at least one Krebs cycle precursor salt as active ingredient, and a coating comprising at least one coating agent, the Krebs cycle precursor salts being selected from fumarates, malates, citrates, alpha-ketoglutarate, succinyl-coenzyme A, succinates and oxaloacetate,
and
- a second pharmaceutical formulation for oral use in the form of at least one mini-tablet, the mini-tablet having a size comprised within a range from 2 to 25 mm, and said mini-tablet being constituted by a core comprising at least one bicarbonate salt as active ingredient and at least one sustained-release matrix, and a coating comprising at least one coating agent, the *in vitro* dissolution of the second formulation in a dissolution medium buffered at a given pH within a range between 1.3 and 7, with a dissolution apparatus of type 2, according to the European Pharmacopoeia (Ph. Eur.) 2.9.3 "*Dissolution test for solid dosage forms*", taking place at a pH-independent kinetics.

2. Composition according to the preceding claim, such that it comprises from 30 to 70% of the first formulation and from 70 to 30% of the second formulation, by weight relative to the total weight of the composition.

3. Composition according to any one of the preceding claims, such that the first formulation comprises from 40% to 80%, preferably from 50 to 70%, by weight of Krebs cycle precursor salt based on the total weight of the first formulation and such that the second formulation comprises from 40% to 80%, preferably from 50 to 80%, by weight bicarbonate salt based on the total weight of the second formulation.

4. Composition according to any one of the preceding claims, such that the Krebs cycle precursor salt is a citrate salt selected from potassium citrate, sodium citrate and magnesium citrate, and more preferably the citrate salt is potassium citrate.

5. Composition according to any one of the preceding claims, such that the first formulation comprises from 0.01% to 5%, preferably from 0.01% to 2 % by weight, even more preferably from 1.4 to 2.5%, coating agent of the microtablet of the first formulation relative to the total weight of the first formulation.

6. Composition according to any one of the preceding claims, such that the coating agent of the microtablet of the first formulation is selected from alginates, carboxyvinyl polymers, sodium salts of carboxymethyl cellulose, cellulose derivatives including the polymers hydroxypropyl methylcellulose, hydroxypropyl ethylcellulose, hydroxypropyl cellulose, hydroxyethyl cellulose, methyl cellulose, ethyl cellulose, xanthan gum and polyethylene oxide, waxes of the paraffin wax, beeswax or carnauba wax type, copolymers of ammonium methacrylate of type A and B as described in the European Pharmacopoeia, and polyacrylates with dispersion of about 30% as described in the European Pharmacopoeia; and preferably the coating agent is a polymer of ethylcellulose.

7. Composition according to any one of the preceding claims, such that the bicarbonate salt is selected from potassium bicarbonate, sodium bicarbonate and magnesium bicarbonate, and preferably the bicarbonate salt is potassium bicarbonate.

8. Composition according to any one of the preceding claims, such that the second formulation comprises from 1% to 20%, preferably from 1.5% to 3% by weight of coating agent of the second-formulation mini-tablet, relative to the total weight of the second formulation.

9. Composition according to any one of the preceding claims, such that the coating agent of the second-formulation mini-tablet is selected from alginates, carboxyvinyl polymers, sodium salts of carboxymethyl cellulose, cellulose derivatives including the polymers hydroxypropyl methylcellulose, hydroxypropyl ethylcellulose, hydroxypropyl cellulose, hydroxyethyl cellulose, methyl cellulose, ethyl cellulose, xanthan gum and polyethylene oxide, waxes of the paraffin wax, beeswax or carnauba wax type, copolymers of ammonium methacrylate of type A and B as described in the European Pharmacopoeia, and polyacrylates with dispersion of about 30% as described in the European Pharmacopoeia; and preferably the coating agent is a polymer of ethylcellulose.

10. Composition according to any one of the preceding claims, such that the second formulation comprises from 10% to 30%, preferably from 15 to 25% by weight sustained-release matrix of the second-formulation mini-tablet, relative to the total weight of the second formulation, said sustained-release matrix of the second formulation being preferably selected from alginates, carboxyvinyl polymers, sodium salts of carboxymethyl cellulose, cellulose derivatives including the polymers hydroxypropyl methylcellulose, hydroxypropyl ethylcellulose, hydroxypropyl cellulose, hydroxyethyl cellulose, methyl cellulose, ethyl cellulose, and polyacrylates with dispersion of about 30% as described in the European Pharmacopoeia, and preferably the sustained-release matrix is a hydroxypropyl methylcellulose.

11. Composition according to any one of the preceding claims, such that the second formulation is able to release the bicarbonate salt *in vitro* in a dissolution medium of solution buffered at pH 1.3 with a dissolution apparatus of type 2, according to the European Phamacopoeia (Ph. Eur.) 2.9.3 *"Dissolution test for solid dosage forms",* at a rate of at most 50% in 4 hours, at most 75% in 6 hours, and at most 90% in 8 hours.

12. Composition according to any one of the preceding claims, such that the first formulation comprises from 55% to 70% of potassium citrate, from 20 to 30% of microcrystalline cellulose, from 0.02% to 2% of magnesium stearate, from 0.01% to 1% of glyceryl behenate and from 1 to 3% of ethyl cellulose, relative to the total weight of the first formulation.

13. Composition according to any one of the preceding claims, such that the second formulation comprises from 60% to 70% of potassium bicarbonate, from 15 to 25% of hypromellose, from 7 to 17% of microcrystalline cellulose, from 1 to 3% of glyceryl behenate, from 0.01% to 1% of magnesium stearate, and from 1.5 to 3% of ethyl cellulose, relative to the total weight of the second formulation.

14. Composition according to any one of the preceding claims, for use as a medicament.

15. Composition according to any one of claims 1 to 15, for use as a medicament for the treatment and/or prevention of cystinuria.
